(19) Europäisches Patentamt
European Patent Office
Office européen des brevets

(11) **EP 3 362 790 B1**

(12) **EUROPÄISCHE PATENTSCHRIFT**

(45) Veröffentlichungstag und Bekanntmachung des Hinweises auf die Patenterteilung:
**24.07.2019   Patentblatt 2019/30**

(21) Anmeldenummer: **16763031.8**

(22) Anmeldetag: **08.09.2016**

(51) Int Cl.:
***G01N 33/22*** *(2006.01)*

(86) Internationale Anmeldenummer:
**PCT/EP2016/071198**

(87) Internationale Veröffentlichungsnummer:
**WO 2017/063795 (20.04.2017 Gazette 2017/16)**

(54) **VERFAHREN ZUM BESTIMMEN VON EIGENSCHAFTEN EINES KOHLENWASSERSTOFFHALTIGEN GASGEMISCHES**

METHOD FOR DETERMINING PROPERTIES OF A HYDROCARBON-CONTAINING GAS MIXTURE

PROCÉDÉ PERMETTANT DE DÉTERMINER DES PROPRIÉTÉS D'UN MÉLANGE GAZEUX CONTENANT DES HYDROCARBURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.10.2015   DE 102015117468**

(43) Veröffentlichungstag der Anmeldung:
**22.08.2018   Patentblatt 2018/34**

(73) Patentinhaber: **Endress+Hauser Flowtec AG
4153 Reinach (CH)**

(72) Erfinder:
• **HUBER, Christof
3007 Bern (CH)**
• **BADARLIS, Anastasios
4127 Birsfelden (CH)**

(74) Vertreter: **Andres, Angelika Maria
Endress+Hauser (Deutschland) AG+Co. KG
PatServe
Colmarer Strasse 6
79576 Weil am Rhein (DE)**

(56) Entgegenhaltungen:
**EP-A1- 2 993 472     WO-A1-2015/074833
DE-B1- 2 928 739     GB-A- 2 296 091**

• ULRICH BONNE ET AL: "Actuation-based microsensors; Actuation-based microsensors", SMART MATERIALS AND STRUCTURES, IOP PUBLISHING LTD., BRISTOL, GB, Bd. 10, Nr. 6, 1. Dezember 2001 (2001-12-01), Seiten 1185-1195, XP020071574, ISSN: 0964-1726, DOI: 10.1088/0964-1726/10/6/307

**Beschreibung**

Die vorliegende Erfindung betrifft ein Verfahren zum Bestimmen von Eigenschaften eines kohlenwasserstoffhaltigen Gasgemisches und eine Vorrichtung dafür. Die relevanten Gasgemische sind insbesondere Erdgas oder Biogas.

Erdgas ist ein fossiler Energieträger. Es hat eine lagerstättenabhängige Zusammensetzung. Der Hauptbestandteil von Erdgas ist Methan, mit einem molaren Anteil von beispielsweise 75 % bis 99 %. Häufig enthält Erdgas auch größere Anteile an Ethan (1 % bis 15 %), Propan (1 % bis 10 %), Butan und Ethen. Weitere Nebenbestandteile sind Schwefelwasserstoff, Stickstoff, Kohlenstoffdioxid und Wasserdampf.

Biogas ist ein brennbares Gasgemisch unterschiedlicher Zusammensetzung, das durch Vergärung von Biomasse jeder Art entsteht. Es enthält im Rohzustand insbesondere Methan (bis zu 60%) und Kohlenstoffdioxid als Hauptkomponenten. Weiterhin sind Stickstoff, Sauerstoff, Schwefelwasserstoff, Wasserdampf und Ammoniak enthalten. Schwefelwasserstoff und Ammoniak müssen vor dem Verbrennen bzw. vordem Einspeisen ins Erdgasnetz entfernt werden. Ebenso ist es vorteilhaft Kohlenstoffdioxid vor der Verbrennung abzuscheiden.

Um Verbrennungsprozesse zuverlässig steuern zu können, ist es erforderlich, den aktuellen Energiegehalt eines Gasgemischs, das gerade einem Prozess zugeführt wird, schnell zu erkennen. Das Patent DE 29 28 739 B1 offenbart ein Verfahren zum Bestimmen des Energiegehalts bzw. des Wobbe-Indexes eines Brenngases auf Basis von dessen Dichte und Viskosität wobei die Viskosität aufgrund eines Strömungswiderstands des Gases bei einem definierten Volumenstrom ermittelt wird.

Bonne et al. beschreiben in "Actuation-based microsensors", Smart Materials and Structures, Vol. 10 (2001) Seiten 1185-1195 Mikrosensoren zur Bestimmung der Zusammensetzung mehrkomponentiger Gase.

Die Patentschrift DE 69 231 977 T2 und US 5 311 447 A1 offenbaren Verfahren zur Bestimmung des Energiegehalts eines Gasgemischs aus der Wärmeleitfähigkeit, der Wärmekapazität, der optischen Absorption und der Viskosität des Gasgemischs.

Eine technisch relevantere Charakterisierung des Energiegehalts dient der Wobbe-Index W, welcher definiert ist als der Quotient aus dem Energiegehalt pro Volumeneinheit H und der Wurzel aus der relativen Dichte. Die relative Dichte ist der Quotient aus der Dichte $\rho$ des Brenngases und der Dichte trockener Luft $\rho_0$ unter gleichen Druck- und Temperaturbedingungen:

$$W = \frac{H}{\sqrt{\frac{\rho}{\rho_0}}}$$

Gasgemische mit gleichem Wobbe-Index können beim Betrieb eines Brenners ohne weiteres ausgetauscht werden.

Die Internationale Norm ISO 6976 legt Verfahren zur Berechnung von Brennwert, Heizwert, Dichte, relativer Dichte und Wobbeindex von trockenen Erdgasen, Erdgasaustauschgasen und sonstigen Brenngasen fest, wenn die Zusammensetzung des Gases in Stoffmengenanteilen bekannt ist.

Veröffentlichung GB 2 296 091 A beschreibt ein Verfahren zur Bestimmung des Brennwerts und des Wobbe-Indexes eines Gasgemischs auf Basis von dessen Wärmeleitfähigkeit, Dichte, Viskosität, und Schallgeschwindigkeit. Die Viskosität wird auf Standardtemperatur und Standarddruck korrigiert. Eine Kontrolleinheit speichert Referenzdaten die auf Standardtemperatur und Standarddruck korrigiert sind. Die MEMS AG bietet unter der Bezeichnung Gas QS einen Sensor an, der auf Basis der Wärmeleitfähigkeit, der Wärmekapazität und der Dichte eines Gasgemischs dessen Brennwert bzw. Wobbe-Index bestimmt.

Die Messung der oben genannten optischen Absorption oder der thermischen Parameter wie Wärmeleitfähigkeit und Wärmekapazität sind jedoch aufwändig.

Es besteht daher Bedarf an einem einfachen, robusten und zuverlässigen Verfahren zur Bestimmung von Eigenschaften eines Gasgemisches, insbesondere dessen Energiegehalt bzw. Wobbe-Index. Es ist daher die Aufgabe der vorliegenden Erfindung, ein solches Verfahren bereitzustellen. Die Aufgabe wird erfindungsgemäß gelöst durch das Verfahren gemäß dem unabhängigen Patentanspruch 1.

Das erfindungsgemäße Verfahren zum Bestimmen von Eigenschaften eines kohlenwasserstoffhaltigen Gasgemisches, welches insbesondere Erdgas oder Biogas aufweist, umfasst:

Strömen Lassen des Gasgemischs durch eine Messanordnung;

Bestimmen eines druck- und temperaturabhängigen Viskositätsmesswerts, eines zugehörigen Temperaturmesswerts und eines zugehörigen Druckmesswerts des strömenden Gasgemischs;

Ermitteln eines ersten Werts einer den Energiegehalt des strömenden Gasgemischs charakterisierenden ersten Größe auf Basis des Viskositätsmesswerts, des zugehörigen Temperaturmesswerts, und des zugehörigen Druckmesswerts, wobei die den Energiegehalt charakterisierende erste Größe der Wobbe Index oder der Brennwert des strömenden Gasgemischs ist, wobei das Ermitteln des ersten Werts der ersten Größe insbesondere auf Basis einer Korrelationsrechnung erfolgt, und Bestimmen eines zweiten Werts einer den Energiegehalt des strömenden Gasgemischs charakterisierenden zweiten Größe auf Basis eines aktuellen Dichtemesswerts oder eines aktuellen Schallgeschwindigkeitsmesswert des strömenden Gasgemischs, bei dem zugehörigen Druckmesswert und dem zugehörigen Temperaturmesswert ohne Verwendung eines Viskositätsmesswerts, wobei die den Energiegehalt charakterisierende zweite Größe der Wobbe Index oder der Brennwert des strömenden Gasgemischs ist, wobei das ermitteln des zweiten Werts der zweiten Größe insbesondere auf Basis einer Korrelationsrechnung erfolgt.

In einer Weiterbildung der Erfindung umfasst das Verfahren weiterhin:

entweder, sofern die erste Größe und die zweite Größe gleich sind, Ermitteln einer Abweichung zwischen dem ersten Wert und dem zweiten Wert; und Ermitteln der Summe des Gehalts von Kohlenstoffdioxid und Stickstoff auf Basis der ermittelten Abweichung, oder, sofern die erste Größe und die zweite Größe verschieden sind, Umrechnen des ersten Werts oder des zweiten Werts in einen entsprechenden Wert der anderen Größe, Ermittelten einer Abweichung zwischen dem umgerechneten Wert, und dem ursprünglich in der anderen charakterisierenden Größe vorliegenden Wert; und Ermitteln der Summe des Gehalts von Kohlenstoffdioxid und Stickstoff auf Basis der ermittelten Abweichung.

In einer Weiterbildung der Erfindung ist die erste Größe der Wobbe Index des strömenden Gasgemischs.
Nach dieser Weiterbildung der Erfindung umfasst das Verfahren zum Bestimmen von Eigenschaften eines kohlenwasserstoffhaltigen Gasgemisches, welches insbesondere Erdgas oder Biogas aufweist: Strömen Lassen des Gases durch eine Messanordnung; Bestimmen eines druck- und temperaturabhängigen Viskositätsmesswerts, eines zugehörigen Temperaturmesswerts und eines zugehörigen Druckmesswerts des strömenden Gases; und Ermitteln des Wobbe-Indexes des strömenden Gases auf Basis des Viskositätsmesswerts, des zugehörigen Temperaturmesswerts, und des zugehörigen Druckmesswerts, wobei das Ermitteln des Wobbe-Indexes insbesondere auf Basis einer Korrelationsrechnung erfolgt.
Der Wobbe Index ist deshalb als den Energiegehalt charakterisierende erste Größe bevorzugt, weil die Korrelation zwischen dem Wobbe Index und der Viskosität besser ist als die Korrelation zwischen dem Brennwert und der Viskosität.
In einer Weiterbildung der Erfindung ist die zweite Größe der Brennwert des strömenden Gasgemischs.
In einer Weiterbildung der Erfindung wird der als Wobbe Index vorliegende erste Wert in einen Brennwert umgerechnet, insbesondere durch Multiplikation des Wobbe-Indexes mit der Wurzel des spezifischen Gewichts des Gasgemischs.

Damit können auf Basis der Viskosität ermittelte Werte für den Wobbe Index und den Brennwert bereitgestellt werden, beispielsweise zum Steuern der Brenngaszufuhr eines Brenners oder zum Ermitteln eines Energieverbrauchs.
In einer Weiterbildung der Erfindung geht dem Ermitteln eines Werts des Wobbe-Indexes als erste Größe das Ermitteln eines Standardviskositätswerts des strömenden Gasgemischs voraus, den das strömende Gasgemisch bei einer Standardtemperatur und einem Standarddruck aufweisen würde, auf Basis des Viskositätsmesswerts, des zugehörigen Temperaturmesswerts und des zugehörigen Druckmesswerts, wobei das Ermitteln des Wobbe-Indexes des strömenden Gasgemischs auf Basis des Standardviskositätswerts des Gasgemischs erfolgt.
In einer Weiterbildung der Erfindung wird zunächst das spezifische Gewicht des Gasgemischs auf Basis eines aktuellen Dichtemesswerts oder eines aktuellen Schallgeschwindigkeitsmesswert des Gasgemischs bestimmt wird, und wobei dann die den Energiegehalt des strömenden Gasgemischs charakterisierenden zweite Größe, insbesondere der Brennwert, auf Basis des spezifischen Gewichts ermittelt wird, wobei insbesondere das spezifische Gewicht des Gasgemischs im Verhältnis zu trockener Luft bei der Standardtemperatur und dem Standarddruck bestimmt wird.
In einer Weiterbildung der Erfindung erfolgt die Bestimmung der Viskosität und ggf. der Dichte des Gasgemischs, mittels eines vibronischen Sensors, wobei der vibronische Sensor ein MEMS-Sensor ist, welcher mindestens ein durchströmtes, schwingendes Messrohr und/oder mindestens einen vom strömenden Gasgemischs umgebenen Oszillator, insbesondere in Form mindestens eines schwingenden Kragträgers oder einer schwingenden Stimmgabel aufweist.
In einer Weiterbildung der Erfindung erfolgt die Bestimmung des Schallgeschwindigkeitsmesswerts mittels Laufzeitmessung zwischen Ultraschallwandlern.
Eine Vorrichtung zum Bestimmen von Eigenschaften eines kohlenwasserstoffhaltigen Gasgemisches, insbesondere mit dem erfindungsgemäßen Verfahren umfasst: eine von dem Gasgemisch durchströmbare Messanordnung, mit einem Temperatursensor, einem Drucksensor und einem vibronischen Sensor zum Bestimmen eines Viskositätsmesswerts und ggf. eines Dichtemesswerts des strömenden Gasgemischs; und eine Auswertungseinheit zum Berechnen von Eigenschaften des strömenden Gasgemischs; wobei die Eigenschaften zumindest den Wobbe-Index des Gases umfassen.
In einer Weiterbildung ist der vibronische Sensor ein MEMS-Sensor, welcher mindestens ein durchströmbares, schwingfähiges Messrohr und/oder mindestens einen vom strömenden Gasgemisch umgebenen Oszillator, insbesondere in Form mindestens eines schwingfähigen Kragträgers oder einer schwingfähigen Stimmgabel aufweist. Ein geeigneter MEMS-Sensor ist beispielsweise in der Offenlegungsschrift DE 10 2014 115 566 A1 beschrieben.

In einer Weiterbildung umfasst die Vorrichtung mindestens einen Ultraschallwandler zur Bestimmung der Schallgeschwindigkeit des strömenden Gasgemischs.

Die Erfindung wird nun anhand der in den Zeichnungen dargestellten Ausführungsbeispiele näher erläutert.

Es zeigt:

Fig. 1a: ein Diagramm der Viskosität und des spezifischen Gewichts reiner Gase als Funktion des Brennwerts;

Fig. 1b: ein Diagramm der Viskosität und der Schallgeschwindigkeit reiner Gase als Funktion des Brennwerts;

Fig. 2a: ein Diagramm der Viskosität und des spezifischen Gewichts reiner Gase als Funktion des Wobbe Indexes;

Fig. 2b: ein Diagramm des Wobbe Indexes verschiedener Gasgemische als Funktion der Viskosität bei verschiedenen Temperaturen und Druckwerten;

Fig. 3a: ein Korrelationsdiagramm zum mittels des erfindungsgemäßen Verfahrens bestimmten Wobbe-Index über dem Wobbe-Index aufgrund der tatsächlichen Zusammensetzung von Gasgemischen;

Fig. 3b: eine statistische Analyse zu den Daten des Korrelationsdiagramms aus Fig. 3a;

Fig. 4a: ein Korrelationsdiagramm zu mittels Korrelationsrechnung mit und ohne Berücksichtigung des Viskositätswerts ermittelten Brennwerten über dem tatsächlichen Brennwert aufgrund der Zusammensetzung von Gasgemischen;

Fig. 4b: ein Korrelationsdiagramm des tatsächlichen Gehalts an Kohlenstoffdioxid und Stickstoff verschiedener Gasgemische über der Differenz zwischen den mittels Korrelationsrechnung mit Berücksichtigung des Viskositätswerts ermittelten Brennwerten und den mittels Korrelationsrechnung ohne Berücksichtigung des Viskositätswerts ermittelten Brennwerten;

Fig. 5a: ein Korrelationsdiagramm zum mittels des erfindungsgemäßen Verfahrens ermittelten Gehalts an Kohlenstoffdioxid und Stickstoff über dem tatsächlichen Gehalt an Kohlenstoffdioxid und Stickstoff von Gasgemischen;

Fig. 5b: eine statistische Analyse zu den Daten des Korrelationsdiagramms aus Fig. 5a;

Fig. 6a: ein Flussdiagramm eines Verfahrens, das nicht gemäß der Erfindung ist;

Fig. 6b: ein Flussdiagramm eines ersten Ausführungsbeispiels des erfindungsgemäßen Verfahrens;

Fig. 6c: ein Flussdiagramm eines zweiten Ausführungsbeispiels des erfindungsgemäßen Verfahrens; und

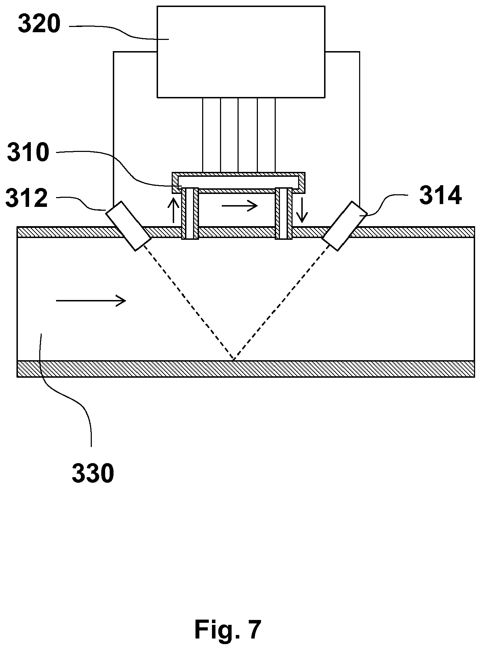

Fig. 7 eine Ausführungsbeispiel einer Vorrichtung zur Durchführung des erfindungsgemäßen Verfahrens.

Die Erfindung beruht auf der statistischen Analyse der physikalischen Eigenschaften von mehreren tausend Erdgasproben in Abhängigkeit von ihrer Zusammensetzung. Die Zusammensetzung wurde mittels Gaschromatographie bestimmt für die ermittelten Zusammensetzungen wurden die physikalischen Eigenschaften der Gasgemische bei verschiedenen Druck- und Temperaturwerten rechnerisch ermittelt. Gleichermaßen wurden die physikalischen Eigenschaften einiger reiner Gase berechnet. Für die rechnerische Ermittlung der physikalischen Eigenschaften wurde ein Programm des NIST verwendet, nämlich "Reference Fluid Thermodynamic and Transport Properties Database", kurz REFPROP, Version 9.1, welches unter der Adresse http://www.nist.gov/srd/nist23.cfm zugänglich ist. Eine experimentelle Bestimmung der physikalischen Größen ist gleichermaßen möglich, bedeutet aber einen größeren Aufwand. Zu den rechnerisch ermittelten physikalischen Größen gehören:

- Dichte: $\rho(T,p)$
- Spezifisches Gewicht: $SG = \rho(T_{ref}, p_{ref})/\rho_{Luft}(T_{ref}, p_{ref})$
- $SG_{ideal}(T,p)$. $\rho(T,p)/\rho_{Air}(T,p)$
- Z-Faktor: $Z = \rho_{ideal}(T.p)/\rho_{real}(T,p)$
= $\rho(T,p)*(T/T_{ref})*(p_{ref}/p) /\rho(T_{ref},p_{ref})$
- Schallgeschwindigkeit SOS
- Dynamische Viskosität: $\eta(T,p)$
- Brennwert: CV
- Wobbe index: $WI = CV/\sqrt{SG}$

Die auf Basis der obigen Daten durchgeführte Entwicklung des erfindungsgemäßen Verfahrens wird im Folgenden näher erläutert.

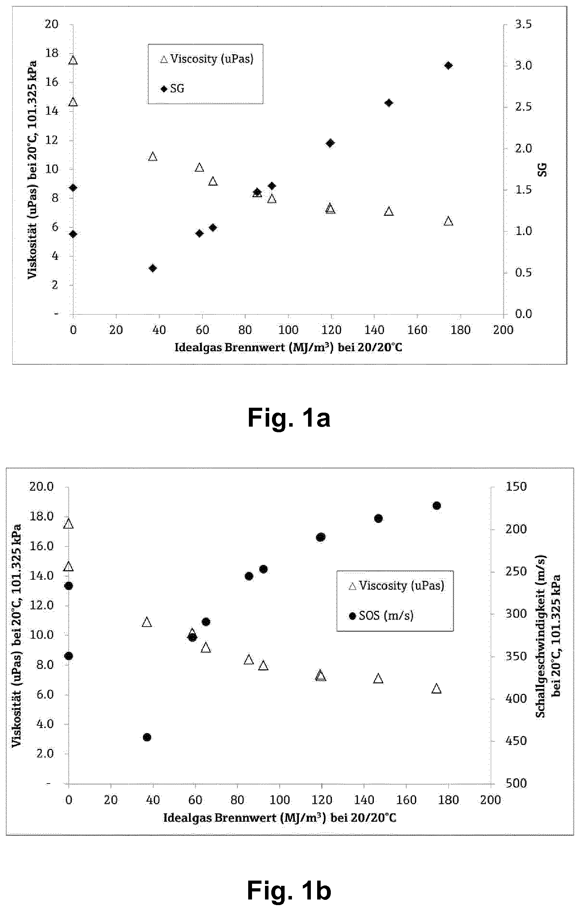

In Fig. 1a sind die dynamische Viskosität und das spezifische Gewicht einiger reiner Gase über deren Brennwert dargestellt. Die Gase sind Methan, Ethan, Propan, n-Butan, iso-Butan, n-Hexan, Ethylen, Propen, Stickstoff und Kohlenstoffdioxid. In Fig. 1b sind wieder die dynamische Viskosität und - in Abwandlung zu Fig. 1a - die Schallgeschwindigkeit der gleichen Gase über deren Brennwert dargestellt. Die Gase sind Methan, Ethan, Propan, n-Butan, iso-Butan, n-Hexan, Ethylen, Propen, Stickstoff und Kohlenstoffdioxid. Es deutet sich in beiden Diagrammen eine brauchbare Korrelation zwischen der dynamischen Viskosität und dem Brennwert der Gase an. Das spezifische Gewicht und die Schallgeschwindigkeit weisen ebenfalls eine deutliche Korrelation zum Brennwert der reinen Brenngase auf, bei Stickstoff und Kohlenstoffdioxid ist diese Korrelation jedoch nicht gegeben, hieraus ergibt sich ein Ansatz den Anteil von Stickstoff und Kohlenstoffdioxid in einem Gasgemisch zu bestimmen, wie weiter unten erläutert wird. Zunächst jedoch zur Bestimmung des Wobbe Indexes und des Brennwerts.

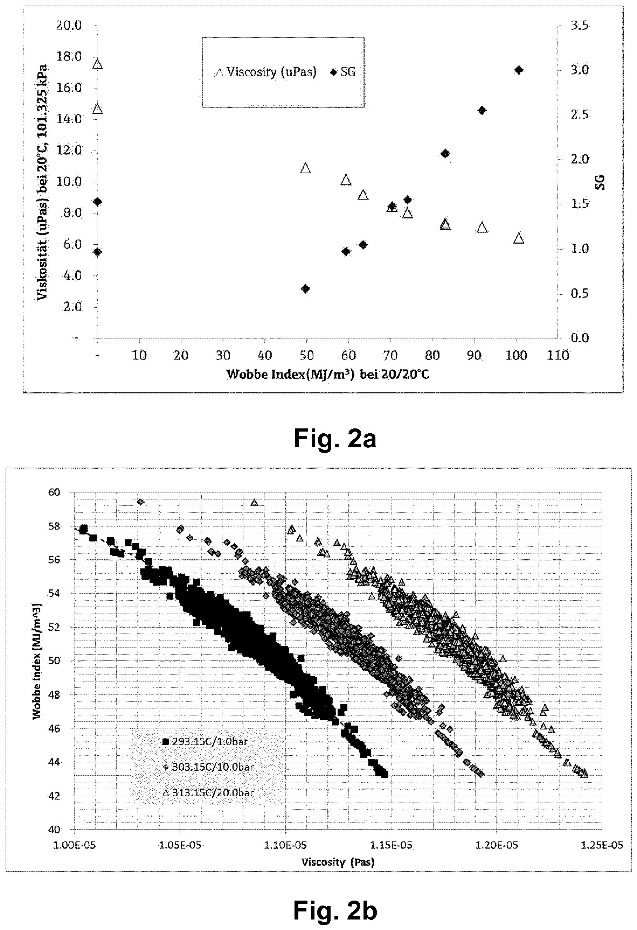

In Fig. 2a sind die dynamische Viskosität und das spezifische Gewicht der gleichen Gase wie in Fign. 1a und 1b über deren Wobbe Index dargestellt. In Kenntnis der Fign. 1a und 1b überrascht es nicht, dass die Viskosität wiederum eine brauchbare Korrelation zum Wobbe Index der gesamten analysierten Gase zeigt, wobei die Korrelation zwischen dem spezifischen Gewicht und dem Wobbe Index reiner Gase nicht zu Kohlenstoffdioxid und Stickstoff zu extrapolieren sind.

Die Korrelation zwischen Wobbe Index und dynamischer Viskosität ergibt nun die Basis zur Bestimmung des Wobbe Indexes auf Basis der Viskosität, wie in Fig. 2b für Gasgemische bei verschiedenen Druck- und Temperaturwerten dargestellt ist. Der Wobbe Index ist beispielsweise als Polynom der Viskosität darstellbar, wobei die Koeffizienten des Polynoms eine Druck- und Temperaturabhängigkeit aufweisen. Das Polynom sollte vorzugsweise mindestens zweiten Grades sein, wobei derzeit ein Polynom dritten Grades bevorzugt ist. Grundsätzlich sind jedoch auch polynome höherer Ordnung zur Bestimmung des Wobbe Indexes möglich, beispielsweise vierter, fünfter oder sechster Ordnung. Zur Implementierung des erfindungsgemäßen Verfahrens ist die Druck- und Temperaturabhängigkeit der Koeffizienten zu bestimmen, um anhand eines bei einem Druck- und Temperaturwert vorliegenden Viskositätswert eines Gasgemisches den Wobbe Index zu bestimmen.

Gleichermaßen kann zunächst aus einem aktuellen Viskositätswert $\eta(T,p)$ bei einem gegebenen Druck p und einer gegebenen Temperatur T zunächst eine Viskosität bei Referenzbedingungen $\eta(STP)$ bestimmt werden, wobei dann der Wobbe Index auf Basis der Viskosität bei Referenzbedingungen zu berechnen ist. Die Viskosität bei Referenzbedingungen $\eta(STP)$ ist aus einem aktuellen Viskositätswert beispielsweise zu berechnen als:

$$\eta(STP) = \eta(T,p)\,/C_\eta$$

Hierbei ist der Kompensationsfaktor $C_\eta$ eine Funktion von Druck und Temperatur, die beispielsweise folgendermaßen gegeben ist:

$$C_\eta = C_0(SG) + C_1\,(SG) \cdot p + C_2\,(SG) \cdot T$$

$$C_0(SG) = a_1 {}^* SG^{\wedge}2 + b_1 {}^* SG + c_1$$

$$C_1(SG) = a_2 {}^* SG^{\wedge}2 + b_2 {}^* SG + c_2$$

$$C_2(SG) = a_3 {}^* SG^{\wedge}2 + b_3 {}^* SG + c_3$$

Hierbei sind die $a_i$, $b_i$, $c_i$ (i = 1, 2, 3) Koeffizienten die vom spezifischen Gewicht SG des Gasgemischs abhängen, für die vorliegende Erfindung wurden die Koeffizienten für die Kompensationsfaktoren anhand der Eigenschaften von mehr als sechstausend Erdgasproben über einen Temperaturbereich zwischen 20°C und 40°C und einem Druckbereich zwischen 1 und 20 bar bestimmt. Der Standardfehler des Kompensationsfaktors beträgt hier weniger als 0,1% und der maximale Fehler weniger als , 8%.

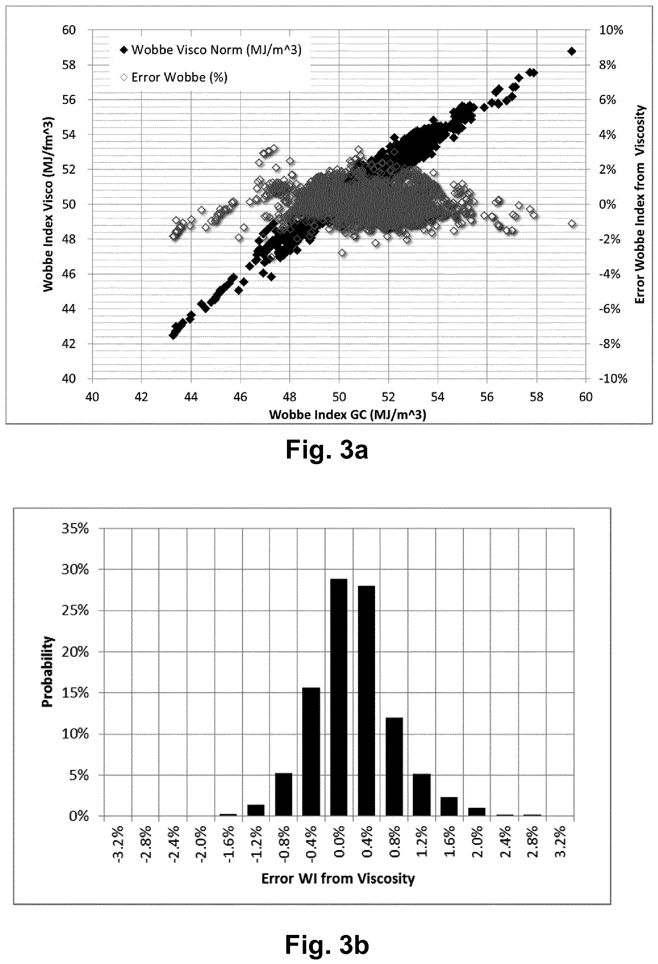

Fig. 3a stellt nun den mit dem erfindungsgemäßen Verfahren ermittelten Wobbe Index auf Basis der Viskosität bei Standardbedingungen $\eta(STP)$ über dem tatsächlichen Wobbe Index für tausende von Erdgasproben dar, wobei die Viskosität bei Standardbedingungen $\eta(STP)$ anhand des obigen Modells aus einer Viskosität $\eta(T,p)$ für beliebige Druck und Temperaturwerte ermittelt wurde. Zudem ist der relative Fehler des erfindungsgemäßen Wobbe Index bezogen auf den tatsächlichen Wobbe Index dargestellt. Eine Verteilung der relativen Fehler des erfindungsgemäß bestimmten Wobbe Index im Verhältnis zum tatsächlichen Wobbe Index ist in Fig. Fig. 3b dargestellt. Demnach beträgt das $3\sigma$ Intervall des Wobbe Index Fehlers auf Basis des erfindungsgemäßen Verfahrens etwa $\pm 3\%$. Dies ist ein hinreichend gutes Ergebnis, um mit dem erfindungsgemäßen Verfahren ein Brenngas zu charakterisieren, das beispielsweise einem Brenner zugeführt wird.

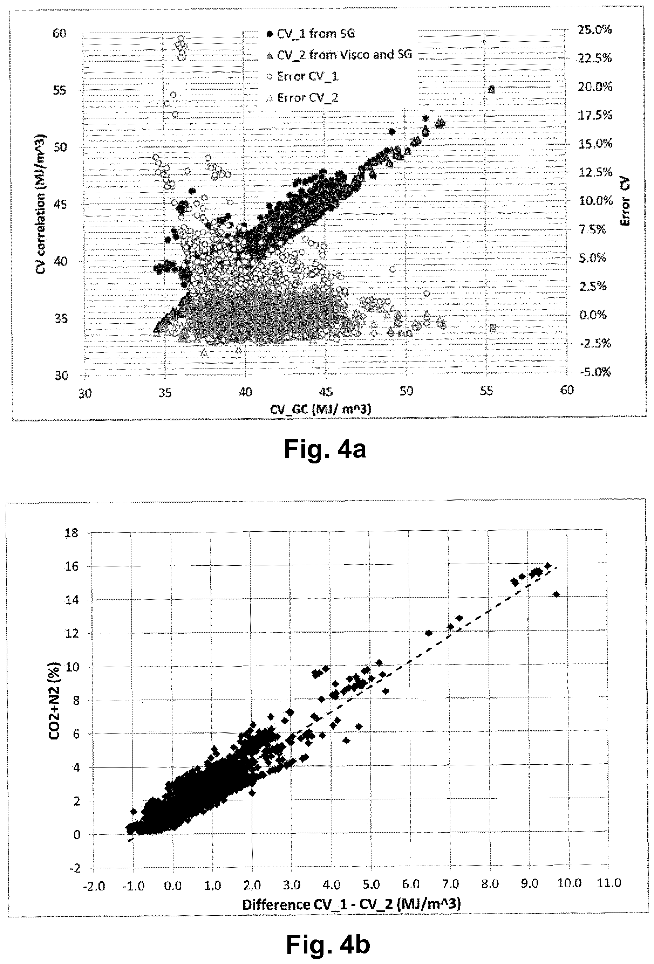

Aus dem erfindungsgemäß auf Basis einer Viskositätsmessung ermittelten Wobbe Index eines Gasgemischs lässt sich durch Multiplikation mit der Wurzel aus dessen spezifischem Gewicht dessen Brennwert bestimmen. Andererseits zeigt der Brennwert eines Gasgemischs eine gute Korrelation zu dessen spezifischem Gewicht, solange die Inertgase Stickstoff und Kohlenstoffdioxid keine Rolle spielen. Kommen diese Komponenten hinzu, gilt die Korrelation nicht mehr. Dieser Sachverhalt ist in Fig. 4a dargestellt. Fig. 4a zeigt mittels obiger Korrelationsverfahren ermittelte Brennwerte von Gasgemischen über den tatsächlichen Brennwerten aufgetragen. Gleichermaßen sind die relativen Fehler, jeweils bezogen auf den tatsächlichen Brennwert dargestellt. Das Diagramm zeigt, dass beide Korrelationsverfahren bei hohen Brennwerten recht gut übereinstimmende und weitgehend korrekte Ergebnisse liefern, und dass bei kleinen Brennwerten, die gewöhnlich mit einem größeren Anteil an Stickstoff und Kohlenstoffdioxid, kurz Inertgasanteil, einhergehen, die Korrelationsrechnung nur auf Basis des spezifischen Gewichts ohne Berücksichtigung der Viskosität abweichende und falsche Ergebnisse für den Brennwert liefert. Es treten Fehler von über 20% auf. Wie bereits angedeutet im Zusammenhang von Fig. 1a, ist ein Ansatz gegeben, aus der Not eine Tugend zu machen. Dies wird nun anhand von Fig. 4b erläutert.

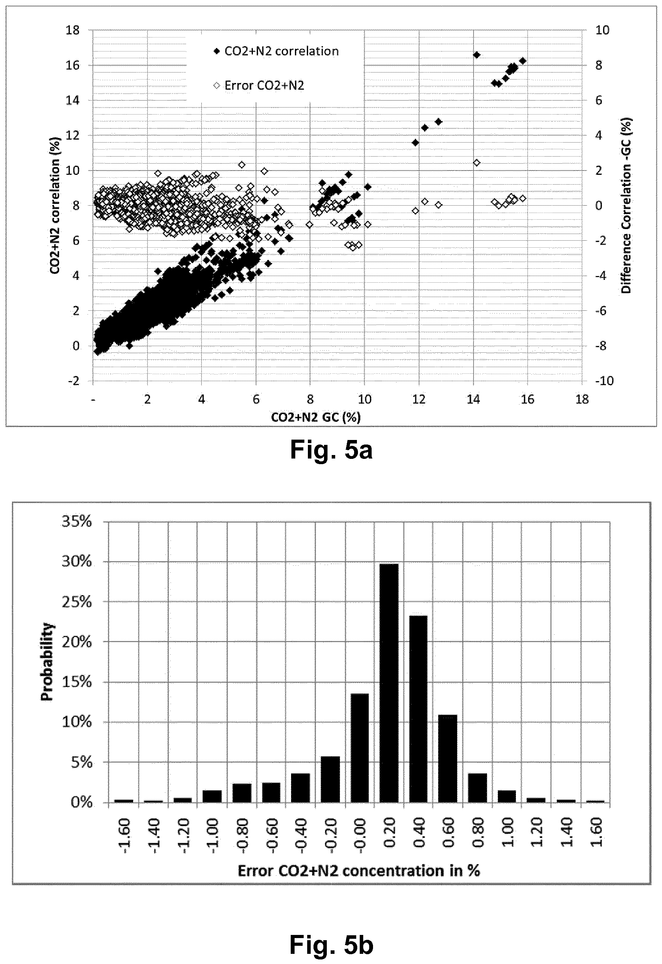

Fig. 4b zeigt den tatsächlichen Inertgasanteil der mehreren tausend untersuchten Erdgasproben über der Differenz zwischen einem ersten durch Korrelationsrechnung ermittelten Brennwert und einem durch Korrelationsrechnung ermittelten zweiten Brennwert, wobei der erste Brennwert nur auf Basis des spezifischen Gewichts ohne Berücksichtigung der Viskosität ermittelt wurde, und wobei der zweite Brennwert über den viskositätsabhängigen Wobbe Index und das spezifische Gewicht ermittelt wurde. Der Inertgasanteil zeigt eine brauchbare Korrelation zur obigen Brennwertdifferenz. Demnach lässt sich der Inertgasanteil als Funktion der Brennwertdifferenz mittels Korrelationsrechnung bestimmen. Mit welcher Genauigkeit dieser Ansatz tatsächlich zur Bestimmung des Inertgasanteils taugt, wird anhand der Fign. 5a und 5b ersichtlich.

Fig. 5a zeigt den erfindungsgemäß mittels Korrelationsrechnung als Funktion der Brennwertdifferenz bestimmten Inertgasanteil über dem tatsächlichen mittels Gaschromatographie bestimmten Inertgasanteil der mehreren tausend untersuchten Erdgasproben. Es zeigt sich eine hervorragende Übereinstimmung. Eine statistische Auswertung der Abweichung zwischen dem erfindungsgemäß bestimmten Inertgasanteil und dem tatsächlichen Inertgasanteil ist in Fig. 5b dargestellt. Der Fehler des Inertgasgehalts hat demnach einen $3\sigma$-Wert von etwa $\pm1.5\%$. Dies ist eine hinreichende Genauigkeit für die Analyse eines Brenngases, beispielsweise bei der Brennersteuerung.

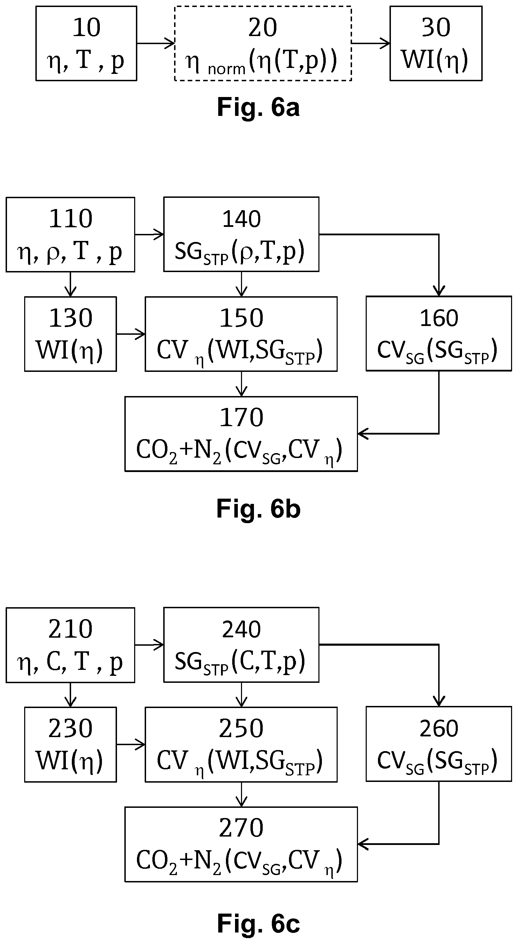

Zusammenfassend werden nun die in den Fign. 6b und 6c dargestellten Flussdiagramme von Ausführungsbeispielen des erfindungsgemäßen Verfahrens vorgestellt.

Das in Fig. 6a dargestellte Verfahren nicht gemäß der Erfindung umfasst in einem Schritt 10 das Erfassen eines Viskositätsmesswerts $\eta$, eines Temperaturmesswerts T und eines Druckmesswerts p des strömenden Gasgemischs, wobei die genannten Messwerte möglichst gleichzeitig zu erfassen sind und die dazu erforderlichen Sensoren vorzugsweise möglichst nahe beieinander angeordnet sind, so dass die Messwerte ein Wertetupel des Gasgemisches in einem thermodynamischen Zustand bilden. Die Messung der Viskosität erfolgt beispielsweise mit einem vibronischen Sensor, insbesondere einem schwingenden Cantilever, der von dem Gasgemisch umgeben ist. Optional kann in einem Schritt 20 aus dem aktuellen Viskositätsmesswert bei einem gegebenen Druck p und einer gegebenen Temperatur T zunächst ein Visositätsmesswert bei Standardbedingungen bestimmt werden. Schließlich wird in einem Schritt 30 auf Basis eines Viskositätswerts, sei es der direkt gemessene Viskositätsmesswert oder der Viskosität bei Standardbedingungen, der Wobbe Index des Gasgemisches bestimmt, wie oben im Zusammenhang mit Fig. 2b erläutert wurde.

Das in Fig. 6b gezeigte Ausführungsbeispiel des erfindungsgemäßen Verfahrens umfasst in einem Schritt 110 das Erfassen eines Viskositätsmesswerts $\eta$, eines Dichtemesswerts $\rho$, eines Temperaturmesswerts T und eines Druckmesswerts p des strömenden Gasgemischs. Für die Messwerterfassung gelten die gleichen Bedingungen wie beim obigen Verfahren nicht gemäß der Erfindung. Die Dichte des Gasgemischs kann ebenfalls mit dem vibronischen Sensor bestimmt werden, da dessen Resonanzfrequenz von der Dichte abhängt.

In einem Schritt 130 wird auf Basis eines Viskositätswerts, sei es der direkt gemessene Viskositätsmesswert oder eine daraus abgeleitete Viskosität bei Standardbedingungen, der Wobbe Index des Gasgemisches bestimmt. In einem Schritt 140 wird aus dem Dichtemesswert, sowie dem zugehörigen Druck- und Temperaturmesswerten das spezifische Gewicht SG des Gasgemischs bestimmt. Aus dem spezifischen Gewicht und dem viskositätsabhängigen Wobbe Index wird in einem Schritt 150 ein erster Wert $CV_\eta$ für den Brennwert ermittelt. Weiterhin wird in einem Schritt 160 nur anhand des spezifischen Gewichts, welches mit dem Brennwert korreliert, solange keine Inertgase vorhanden sind, wie anhand von Fig. 1a erläutert wurde, ein zweiter Wert für den Brennwert des Gasgemischs ermittelt. Aus der Differenz zwischen dem ersten Wert für den Brennwert des Gasgemischs und dem zweiten Wert für den Brennwert des Gasgemischs wird der Inertgasanteil bestimmt, wie in Fig. 4b dargestellt ist.

Das in Fig. 6c gezeigte Ausführungsbeispiel des erfindungsgemäßen Verfahrens umfasst in einem Schritt 210 das Erfassen eines Viskositätsmesswerts $\eta$, eines Werts für die Schallgeschwindigkeit C, eines Temperaturmesswerts T und eines Druckmesswerts p des strömenden Gasgemischs. Für die Messwerterfassung gelten die gleichen Bedingungen wie beim obigen Verfahren nicht gemäß der Erfindung. Die Schallgeschwindigkeit C kann beispielsweise mit zwei Ultraschallwandlern, beispielsweise eines Durchflussmessgeräts bestimmt werden, das den Durchfluss des strömenden Gasgemischs erfasst werden.

In einem Schritt 230 wird auf Basis eines Viskositätswerts, sei es der direkt gemessene Viskositätsmesswert oder eine daraus abgeleitete Viskosität bei Standardbedingungen, der Wobbe Index des Gasgemisches bestimmt. In einem Schritt 240 wird aus der Schallgeschwindigkeit, sowie dem zugehörigen Druck- und Temperaturmesswerten das spezifische Gewicht SG des Gasgemischs bestimmt. Aus dem spezifischen Gewicht und dem viskositätsabhängigen Wobbe Index wird in einem Schritt 250 ein erster Wert $CV_\eta$ für den Brennwert ermittelt. Weiterhin wird in einem Schritt 260 nur anhand des spezifischen Gewichts, welches mit dem Brennwert korreliert, solange keine Inertgase vorhanden sind, wie anhand von Fig. 1a erläutert wurde, ein zweiter Wert für den Brennwert des Gasgemischs ermittelt. Aus der Differenz zwischen dem ersten Wert für den Brennwert des Gasgemischs und dem zweiten Wert für den Brennwert des Gasgemischs wird der Inertgasanteil bestimmt, wie in Fig. 4b dargestellt ist.

Das erfindungsgemäße Verfahren operiert weiterhin unter der Annahme, dass das strömende Gas molekularen Wasserstoff, der auch zum Brennwert beitragen würde, allenfalls in geringer Konzentration, beispielsweise nicht mehr als 1 %, insbesondere nicht mehr als 0,1% enthält. Diese Annahme ist für Erdgas und Biogas gerechtfertigt. Wenn das Brenngas höhere Wasserstoffanteile enthält führt dies entsprechend zu systematischen Fehlern.

Das Beispiel einer Vorrichtung zur Durchführung des erfindungsgemäßen Verfahrens umfasst eine von dem Gasgemisch durchstrombare Messzelle 310, in welcher hier nicht im einzelnen dargestellte Sensorelemente, nämlich ein Cantileverschwinger zum Bestimmen der Viskosität und ggf. der Dichte eines Gasgemischs in der Messzelle, ein Drucksensor und ein Temperatursensor angeordnet sind. Die Sensorelemente sind vorzugsweise in MEMS-Technologie realisiert. Die Vorrichtung umfasst weiterhin eine Betriebs- und Auswerteeinheit 320 zum Treiben der Sensorelemente, zum Auswerten von deren Signalen, um die primären Messgrößen, wie Viskosität, Druck, Temperatur und ggf. Dichte zu bestimmen, und zum Ermitteln des Wobbe Index und / oder des Brennwerts und/oder des Inertgasanteils eines die 310 Messzelle durchströmenden Gasgemischs. Die Betriebs- und Auswerteeinheit umfasst hierzu eine Recheneinheit, die kompakt oder modular aufgebaut sein kann, und insbesondere räumlich voneinander getrennte Module umfassen kann. Die Messzelle 310 ist insbesondere in einer Bypassanordnung an eine Gasleitung 330 angeschlossen, wobei ein Volumenstrom des Gasgemischs mittels einer Druckdifferenz über der Messzelle 310, beispielsweise aufgrund einer Blende bzw. einer Venturidüse in der Rohrleitung, oder mittels einer hier nicht dargestellten Pumpe durch die Messzelle 310 getrieben werden kann.

Die Vorrichtung kann weiterhin zwei Ultraschallwandler 312 aufweisen, die beispielsweise an die Gasleitung 330 angeschlossen sind, um den Volumendurchfluss durch die Gasleitung und die Schallgeschwindigkeit des strömenden Gasgemischs, beispielsweise im Laufzeitdifferenzverfahren zu bestimmen.

**Patentansprüche**

1. Verfahren zum Bestimmen von Eigenschaften eines kohlenwasserstoffhaltigen Gasgemisches, welches insbesondere Erdgas oder Biogas aufweist, umfassend:

   Strömen Lassen des Gasgemischs durch eine Messanordnung;
   Bestimmen eines druck- und temperaturabhängigen Viskositätsmesswerts, eines zugehörigen Temperaturmesswerts und eines zugehörigen Druckmesswerts des strömenden Gasgemischs (110; 210);
   Ermitteln eines ersten Werts einer den Energiegehalt des strömenden Gasgemischs charakterisierenden ersten Größe auf Basis des Viskositätsmesswerts, des zugehörigen Temperaturmesswerts, und des zugehörigen Druckmesswerts, wobei die den Energiegehalt charakterisierende erste Größe der Wobbe Index (130; 230) oder der Brennwert des strömenden Gasgemischs ist (150; 250); und
Bestimmen eines zweiten Werts einer den Energiegehalt des strömenden Gasgemischs charakterisierenden zweiten Größe auf Basis eines aktuellen Dichtemesswerts oder eines aktuellen Schallgeschwindigkeitsmesswert des strömenden Gasgemischs (160; 260), bei dem zugehörigen Druckmesswert und dem zugehörigen Temperaturmesswert ohne Verwendung eines Viskositätsmesswerts, wobei die den Energiegehalt charakterisierende zweite Größe der Wobbe Index oder der Brennwert des strömenden Gasgemischs ist.

2. Verfahren nach Anspruch 1, weiterhin umfassend:

   entweder, sofern die erste Größe und die zweite Größe gleich sind, Ermitteln einer Abweichung zwischen dem ersten Wert und dem zweiten Wert; und Ermitteln der Summe des Gehalts von Kohlenstoffdioxid und Stickstoff auf Basis der ermittelten Abweichung (170; 270),
   oder, sofern die erste Größe und die zweite Größe verschieden sind, Umrechnen des ersten Werts oder des zweiten Werts in einen entsprechenden Wert der anderen Größe (150; 250), Ermittelten einer Abweichung zwischen dem umgerechneten Wert, und dem ursprünglich in der anderen charakterisierenden Größe vorliegenden Wert; und
   Ermitteln der Summe des Gehalts von Kohlenstoffdioxid und Stickstoff auf Basis der ermittelten Abweichung (170; 270).

3. Verfahren nach einem der vorhergehenden Ansprüche, wobei die erste Größe der Wobbe Index (130; 230) des strömenden Gasgemischs ist.

4. Verfahren nach einem der vorhergehenden Ansprüche, wobei die zweite Größe der Brennwert des strömenden Gasgemischs (160; 260) ist.

5. Verfahren nach einem der vorhergehenden Ansprüche, wobei der als Wobbe Index vorliegende erste Wert in einen Brennwert (150; 250) umgerechnet wird.

6. Verfahren nach einem der vorhergehenden Ansprüche, wobei dem Ermitteln eines ersten Werts des Wobbe-Indexes als erster Größe das Ermitteln eines Standardviskositätswerts des strömenden Gasgemischs, den das strömende Gasgemisch bei einer Standardtemperatur und einem Standarddruck aufweisen würde, auf Basis des Viskositätsmesswerts, des zugehörigen Temperaturmesswerts und des zugehörigen Druckmesswerts vorausgeht, wobei das Ermitteln des Wobbe-Indexes des strömenden Gasgemischs auf Basis des Standardviskositätswerts des Gasgemischs erfolgt.

7. Verfahren nach einem der vorhergehenden Ansprüche, wobei zunächst das spezifische Gewicht des Gasgemischs auf Basis des aktuellen Dichtemesswerts oder des aktuellen Schallgeschwindigkeitsmesswert des Gasgemischs bestimmt wird, und wobei dann der zweite Wert der den Energiegehalt des strömenden Gasgemischs charakterisierenden zweiten Größe auf Basis des spezifischen Gewichts ermittelt wird.

8. Verfahren nach Anspruch 7, falls abhängig von Anspruch 6, wobei das spezifische Gewicht des Gasgemischs im Verhältnis zu trockener Luft bei der Standardtemperatur und dem Standarddruck bestimmt wird.

9. Verfahren nach einem der vorhergehenden Ansprüche, wobei die Bestimmung der Viskosität und gegebenenfalls der Dichte des Gasgemischs, mittels eines vibronischen Sensors erfolgt, wobei der vibronische Sensor insbesondere ein MEMS-Sensor ist, welcher mindestens ein durchströmtes, schwingendes Messrohr und/oder mindestens einen vom strömenden Gasgemischs umgebenen Oszillator, insbesondere in Form mindestens eines schwingenden Kragträgers oder einer schwingenden Stimmgabel aufweist.

10. Verfahren nach einem der vorhergehenden Ansprüche, wobei die Bestimmung des Schallgeschwindigkeitswerts mittels Laufzeitmessung zwischen Ultraschallwandlern erfolgt.

## Claims

1. Procedure to determine properties of a hydrogenous gas mixture, which particularly contains natural gas or biogas,
comprising the following steps:

   The gas mixture is allowed to flow through a measuring arrangement;
   Determination of a pressure-dependent and temperature-dependent viscosity measured value, an associated temperature measured value and an associated pressure measured value of the flowing gas mixture (110, 210);
   Determination of a first value of a first variable, which characterizes the energy content of the flowing gas mixture, on the basis of the viscosity measured value, the associated temperature measured value, and the associated pressure measured value, wherein the first variable that characterizes the energy content is the Wobbe index (130, 220) or the calorific value of the flowing gas mixture (150, 250); and
Determination of a second value of a second variable, which characterizes the energy content of the flowing gas mixture, on the basis of a current density measured value or a current sound velocity measured value of the flowing gas mixture (160, 260) at the corresponding pressure measured value and the corresponding temperature measured value without using a viscosity measured value, wherein the second variable that characterizes the energy content is the Wobbe index or the calorific value of the flowing gas mixture.

2. Procedure as claimed in Claim 1, further comprising:

   either - if the first variable and the second variable are the same - the determination of a deviation between the first value and the second value; and
   determination of the sum of the content of carbon dioxide and nitrogen on the basis of the deviation determined (170, 270),
   or - if the first variable and the second variable are not the same - conversion of the first value or the second value to a corresponding value of the other variable (150, 250), determination of a deviation between the converted value and the value originally available in the other characteristic variable; and
   determination of the sum of the of the content of carbon dioxide and nitrogen on the basis of the deviation determined (170, 270).

3. Procedure as claimed in one of the previous claims, wherein the first variable is the Wobbe index (130, 230) of the flowing gas mixture.

4. Procedure as claimed in one of the previous claims, wherein the second variable is the calorific value of the flowing gas mixture (160, 260).

5. Procedure as claimed in one of the previous claims, wherein the first value, which is available as a Wobbe index, is converted to a calorific value (150, 250).

6. Procedure as claimed in one of the previous claims, wherein the determination of a first value of the Wobbe index as the first variable is preceded by the determination of a standard viscosity value of the flowing gas mixture which the flowing gas mixture would have at a standard temperature and a standard pressure, on the basis of the viscosity measured value, the associated temperature measured value and the associated pressure measured value, wherein the Wobbe index of the flowing gas mixture is determined on the basis of the standard viscosity value of the gas mixture.

**7.** Procedure as claimed in one of the previous claims, wherein the specific weight of the gas mixture is first determined on the basis of the current density measured value or of the current sound velocity measured value of the gas mixture, and wherein the second value of the second variable that characterizes the energy content of the flowing gas mixture is then determined on the basis of the specific weight.

**8.** Procedure as claimed in Claim 7, if dependent on Claim 6, wherein the specific weight of the gas mixture is determined in relation to dry air at the standard temperature and the standard pressure.

**9.** Procedure as claimed in one of the previous claims, wherein the viscosity and, where applicable, the density of the gas mixture is/are determined using a vibronic sensor, wherein the vibronic sensor is a MEMS sensor, in particular, which has at least a vibrating measuring tube through which fluid flows and/or at least one oscillator surrounded by the flowing gas mixture, particularly in the form of at least a vibrating cantilever or a vibrating tuning fork.

**10.** Procedure as claimed in one of the previous claims, wherein the sound velocity value is determined using a measurement of the transit time between ultrasonic transducers.

## Revendications

**1.** Procédé destiné à la détermination de propriétés d'un mélange gazeux hydrocarboné, lequel contient notamment du gaz naturel ou du biogaz, comprenant :

Mise en écoulement du mélange gazeux à travers un dispositif de mesure ;
Détermination d'une valeur mesurée de viscosité dépendant de la pression et de la température, d'une valeur mesurée de température correspondante et d'une valeur mesurée de pression correspondante du mélange gazeux (110, 210) en écoulement ;
Détermination d'une première valeur d'une première grandeur caractérisant le contenu énergétique du mélange gazeux, sur la base de la valeur mesurée de viscosité, de la valeur mesurée de température correspondante et de la valeur mesurée de pression correspondante, la première grandeur caractérisant le contenu énergétique étant l'indice de Wobbe (130, 220) ou la puissance calorifique du mélange gazeux (150, 250) en écoulement ; et

Détermination d'une deuxième valeur d'une deuxième grandeur caractérisant le contenu énergétique du mélange gazeux, sur la base de la valeur mesurée de densité ou d'une valeur mesurée de vitesse du son du mélange gazeux (160, 260) en écoulement, à la valeur mesurée de pression correspondante et à la valeur mesurée de température correspondante, sans utilisation d'une valeur mesurée de viscosité, la deuxième première grandeur caractérisant le contenu énergétique étant l'indice de Wobbe ou la puissance calorifique du mélange gazeux en écoulement.

**2.** Procédé selon la revendication 1, comprenant en outre :

soit, dans la mesure où la première grandeur et la deuxième grandeur sont égales, détermination d'un écart entre la première valeur et la deuxième valeur ;
détermination de la somme de la teneur en dioxyde de carbone et en azote sur la base de l'écart (170, 270) déterminé,
soit, dans la mesure où la première grandeur et la deuxième grandeur sont différentes, conversion de la première valeur ou de la deuxième valeur en une valeur correspondante de l'autre grandeur (150, 250), détermination d'un écart entre la valeur convertie et la valeur disponible à l'origine dans l'autre grandeur caractéristique ; et
détermination de la somme de la teneur en dioxyde de carbone et en azote sur la base de l'écart (170, 270) déterminé.

**3.** Procédé selon l'une des revendications précédentes, pour lequel la première grandeur est l'indice de Wobbe (130, 230) du mélange gazeux en écoulement.

**4.** Procédé selon l'une des revendications précédentes, pour lequel la deuxième grandeur est la puissance calorifique (160, 260) du mélange gazeux en écoulement.

**5.** Procédé selon l'une des revendications précédentes, pour lequel la première valeur disponible en tant qu'indice de Wobbe est convertie en une puissance calorifique (150, 250).

**6.** Procédé selon l'une des revendications précédentes, pour lequel la détermination d'une première valeur de l'indice de Wobbe en tant que première grandeur est précédée de la détermination d'une valeur de viscosité standard du mélange gazeux en écoulement, valeur de viscosité que présenterait le mélange gazeux en écoulement à une température standard ou à une pression standard, sur la base de la valeur mesurée de viscosité, de la valeur mesurée de température correspondante et de la valeur mesurée de pression correspondante, la détermination de l'indice de Wobbe du mélange gazeux en écoulement étant effectuée sur la base de la valeur de viscosité standard du mélange gazeux.

7. Procédé selon l'une des revendications précédentes, pour lequel le poids spécifique du mélange gazeux est déterminé, dans un premier temps, sur la base de la valeur mesurée de densité actuelle ou de la valeur mesurée de vitesse du son actuelle du mélange gazeux, la deuxième valeur de la deuxième grandeur caractérisant le contenu énergétique du mélange gazeux en écoulement étant ensuite déterminée sur la base du poids spécifique.

8. Procédé selon la revendication 7, si dépendante de la revendication 6, pour lequel le poids spécifique du mélange gazeux est déterminé par rapport à l'air sec à la température standard et à la pression standard.

9. Procédé selon l'une des revendications précédentes, pour lequel la détermination de la viscosité et, le cas échéant, de la densité du mélange gazeux est effectuée au moyen d'un capteur vibronique, le capteur vibronique étant notamment un capteur MEMS, lequel présente au minimum un tube de mesure vibrant, parcouru par le mélange gazeux, et/ou au minimum un oscillateur entouré d'un mélange gazeux en écoulement, notamment sous la forme au minimum d'un support en porte-à-faux ou d'un diapason vibrant.

10. Procédé selon l'une des revendications précédentes, pour lequel la détermination de la valeur de vitesse du son est réalisée au moyen d'une mesure du temps de propagation entre les convertisseurs ultrasonores.

**Fig. 1a**

**Fig. 1b**

**Fig. 2a**

**Fig. 2b**

**Fig. 3a**

**Fig. 3b**

**Fig. 4a**

**Fig. 4b**

**Fig. 5a**

**Fig. 5b**

**Fig. 6a**

**Fig. 6b**

**Fig. 6c**

**320**

**310**

**312**

**314**

**330**

**Fig. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2928739 B1
- DE 69231977 T2
- US 5311447 A1
- GB 2296091 A
- DE 102014115566 A1

**In der Beschreibung aufgeführte Nicht-Patentliteratur**

- **BONNE et al.** Actuation-based microsensors. *Smart Materials and Structures,* 2001, vol. 10, 1185-1195